(19)

Europäisches
Patentamt
European
Patent Office
Office européen
des brevets

(11) **EP 3 778 925 A1**

(12) **EUROPEAN PATENT APPLICATION**
published in accordance with Art. 153(4) EPC

(43) Date of publication:
**17.02.2021 Bulletin 2021/07**

(21) Application number: **19774757.9**

(22) Date of filing: **26.03.2019**

(51) Int Cl.:
*C12Q 1/689* (2018.01)     *C12N 15/11* (2006.01)

(86) International application number:
**PCT/JP2019/013021**

(87) International publication number:
**WO 2019/189266 (03.10.2019 Gazette 2019/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.03.2018   JP 2018058663**

(71) Applicant: **Mitsui Chemicals, Inc.**
**Minato-ku**
**Tokyo 105-7122 (JP)**

(72) Inventors:
• **MORISHIGE, Takashi**
  **Tokyo 105-7122 (JP)**
• **AMANO, Koh**
  **Mobara-shi, Chiba 297-0017 (JP)**
• **YANAI, Hisaaki**
  **Mobara-shi, Chiba 297-0017 (JP)**
• **ENDO, Ayako**
  **Mobara-shi, Chiba 297-0017 (JP)**
• **TSUJI, Kentaro**
  **Mobara-shi, Chiba 297-0017 (JP)**

(74) Representative: **Mewburn Ellis LLP**
**Aurora Building**
**Counterslip**
**Bristol BS1 6BX (GB)**

(54) **BACTERIAL IDENTIFICATION METHOD USING RNA OF SAMPLE BACTERIA, AND KIT THEREFOR**

(57)     The purpose of the present invention is to provide an identification method capable of identifying a large number of bacteria, rather than a limited number of species, without changing the reagents or conditions for identification. Another purpose of the present invention is to provide an identification method that enables high-sensitivity detection and identification of bacteria with only one PCR, reducing the complexity of the operation and the burden on workers.

In the present invention, a method having the following steps is used to identify a bacterium. (1) A step of performing a reverse transcription reaction using a first reaction system consisting of a primer for reverse transcription to prepare a cDNA containing a base sequence for identification of a bacterium of interest, an RNA extracted from the bacterium in a sample, and an enzyme with RNA-dependent DNA polymerase activity to obtain a reaction mixture containing the synthesized cDNA and the primers for the reverse transcription, (2) A step of performing PCR using a second reaction system consisting of the reaction mixture, a primer pair for synthesis of a double-stranded DNA containing the base sequence for identification of the bacterium of interest, and an enzyme with DNA-dependent DNA polymerase activity, provided that in the second reaction system, the concentration of the primer for reverse transcription supplied from the reaction mixture is not less than 0.08nM and not more than 20nM, and (3) A step of detecting the generation of the double-stranded DNA containing the base sequence for identification of the bacterium of interest from the second reaction system after PCR.

EP 3 778 925 A1

**Description**

Technical field

The present invention relates to a rapid identification method for bacteria in a sample and a kit therefor.

Technical background

The number of patients with sepsis, which is a serious systemic infection and the detection and identification of causative microorganisms in the blood is essential for a definitive diagnosis, has increased in recent years with the sophistication of medical care, such as cancer treatment and organ transplantation. Multidrug-resistant organisms, including methicillin-resistant Staphylococcus aureus (MRSA:methicillin-resistant Staphylococcus aureus), are often the causative agents of septicemia from the viewpoint of nosocomial infections, and it is clinically important to detect and identify the causative organisms in the blood as quickly as possible in order to select appropriate antibiotics and save patients (See Patent Document 1).

Against this background, a test system using polymerase chain reaction (PCR) has been investigated as a rapid test method for sepsis. As such a test method, it is known that DNA is extracted from microorganisms and gene amplification is carried out by PCR, etc. using a specific primer pair as a template, and then PCR method that rapidly detects and identifies the causative organism by analyzing the combination of the melting temperature (Tm value) specific to the microorganism or the difference between each Tm value (See Patent Document 2).

In the above PCR method, the extracted DNA is sensitively detected by performing 1st PCR on the template, amplifying the full length of the DNA fragment of interest, and 2nd PCR (nested PCR of the amplified DNA fragment into the template. However, the need for a two-step PCR is a challenge in test time and workability. Especially in the workability, the two PCRs were complicated, and the improvement was necessary for the workload reduction of the worker and total automation of the work.

Bacterial RNA in various clinical, food, environmental, or other experimental samples has also been detected to identify contaminants or pathogens. By targeting several important bacterial species for detection and detecting RNA molecules unique to the bacterial species being detected, specific probes have been developed that enable their detection even in the presence of bacterial species that are not targeted for detection. In a reverse transcription PCR assay method, a method has also been performed in which a cDNA is synthesized by a reverse transcription reaction using an RNA of interest as a template, and a target RNA molecule is detected through amplification of a cDNA in the subsequent PCR (Patent Document 3).

As for the bacterial identification method based on the Tm value, in Patent Document 1, RNA is extracted instead of DNA, and a cDNA prepared based on the obtained RNA can be also utilized, but a detailed examination has not been performed.

Patent Document 3 describes a method for directly detecting a diagnostic RNA from bacteria.

Patent Document 4 describes a method for specific detection of RNA species in a biological sample.

Prior-art document

Patent Application

Patent Document 1: WO2010/082640
Patent Document 2: WO2007/097323
Patent Document 3: JP2012-34705
Patent Document 4: WO2010/054819

Summary of the invention

Problems to be solved by the present invention

Based on the technique disclosed in Patent Document 2, it is possible to detect and identify microorganisms with high sensitivity in a short time.

However, as mentioned above, the above method requires two PCRs, and it is considered that the operation is complicated and not only burdens the worker, but may also be an obstacle to the total automation of the work.

In addition, Patent Document 4 describes a method in which an RNA is reverse-transcribed into a cDNA using a reverse transcriptase, and then a cDNA amplified by PCR is detected.

There is much RNA in bacteria than in DNA.

If considered logically, extracting RNA from bacteria in a sample, preparing cDNA by reverse-transcription reactions using RNA instead of the first-step PCR, and using the prepared cDNA as a template for PCR, can be expected to improve the sensitivity for detecting the bacterium,.

However, according to studies conducted by the present inventors, it has been found that, when a reaction as described in Patent Document 4 is performed, an extra reaction occurs during PCR due to a reverse transcription reaction, and a large number of unnecessary DNA fragments other than the target DNA fragments are obtained, so that virtually no bacterial identification can be achieved.

It is an object of the present invention to provide a method for identifying a bacterium capable of identifying a bacterium without changing a reagent or a condition for identifying a bacterium, and a kit therefor, which is not a limited number of bacteria by targeting an RNA of a bacterium in a sample, and which can identify many bacteria without changing a reagent or a condition for identifying a bacterium.

Further, it is an object of the present invention to provide an identification method and a kit used therefor which can be detected and identified with high sensitivity even by only 1 PCR and can reduce a complicated operation and a burden on an operator.

It is another object of the present invention to find a bacterial identification method necessary for solving the above problem.

As mentioned above, it is evident by the consideration of the inventors that detection and identification of bacteria may not be achieved by the conventional idea of using RNA in a sample alone.

The present inventors have found that too high a concentration of a primer for reverse transcription brought into a PCR step for detecting a target cDNA is a cause when virtually no bacterial identification is possible in the method described in Patent Document 4

The present inventors repeatedly examined this experimental fact as a starting point, and further found, for the first time, that there were new solving problems that were not previously recognized by those skilled in the art. That is, it has been found that when an RNA extracted from a sample is prepared by a reverse transcriptase and a prepared cDNA is subjected to PCR as a template, the generation of a DNA fragment other than a target generated during PCR needs to be suppressed.

This challenge is unknown, where the challenge itself has not been recognized by a skill in the art in the art.

Means for solving the problems

As described above, the inventors have studied diligently to solve a completely novel problem.

As a result, we found that by adjusting the concentration of the reverse transcription primer in the reaction system during the PCR reaction, which is brought in from the reverse transcription reaction, so that the concentration is extremely low than usually thought, the generation of unnecessary DNA fragments is remarkably suppressed, and the bacteria in the sample can be detected and identified by appropriately adjusting the conditions for Tm mapping.

Furthermore, we found that the concentration of the reverse transcription primer in the PCR reaction has an optimal range, and if the concentration of the reverse transcription primer is properly controlled so that it is within this range, it is possible to detect and identify bacteria with high sensitivity, even if the number of bacteria in the sample is very low.

The present invention has been completed on the basis of completely new findings as described above.

That is, the present invention is as follows.

[1] A bacterial identification method characterized by having the following steps (1) to (3):

(1) A step of performing a reverse transcription reaction using a first reaction system comprising a primer for reverse transcription to prepare a cDNA containing a base sequence for identification of a bacterium of interest, an RNA extracted from the bacterium in a sample, and an enzyme with RNA-dependent DNA polymerase activity to obtain a reaction mixture containing the synthesized cDNA and the primers for the reverse transcription,

(2) A step of performing PCR using a second reaction system comprising the reaction mixture, a primer pair for synthesis of a double-stranded DNA containing the base sequence for identification of the bacterium of interest, and an enzyme with DNA-dependent DNA polymerase activity, provided that in the second reaction system, the concentration of the primer for reverse transcription supplied from the reaction mixture is not less than 0.08nM and not more than 20nM, and

(3) A step of detecting the generation of the double-stranded DNA containing the base sequence for identification of the bacterium of interest from the second reaction system after PCR.

[2] The bacterial identification method according to [1] above, characterized in that the steps (1) and (2) are performed sequentially in different reaction vessels or in the same reaction vessel.

[3] The bacterial identification method according to [1] or [2] above, characterized in that the basic sequence for identification of the bacterium of interest is a basic sequence contained in a 16S rDNA of the bacterium and the RNA includes a 16S rRNA of the bacterium in the sample.

[4] The bacterial identification method according to [3] above, characterized in that the primer for reverse transcription consist of a base sequence of SEQ ID NO : 1.

[5] The bacterial identification method according to any one of [1] or [4] above, characterized in that the primer pair is at least one primer pair selected from the group comprising the following (Group A) to (Group C):

(Group A)

- A primer pair 1a: a combination of a forward primer consisting of a base sequence of SEQ ID NO : 2 and a reverse primer consisting of a base sequence of SEQ ID NO : 3,
- A primer pair 2a: a combination of a forward primer consisting of a base sequence of SEQ ID NO : 4 and a reverse primer consisting of a base sequence of SEQ ID NO : 5,
- A primer pair 3a: a combination of a forward primer consisting of a base sequence of SEQ ID NO : 6 and a reverse primer consisting of a base sequence of SEQ ID NO : 7,
- A primer pair 4a: a combination of a forward primer consisting of a base sequence of SEQ ID NO : 8 and a reverse primer consisting of a base sequence of SEQ ID NO : 9,
- A primer pair 5a: a combination of a forward primer consisting of a base sequence of SEQ ID NO : 10 and a reverse primer consisting of a base sequence of SEQ ID NO : 11,
- A primer Pair 6a: a combination of a forward primer consisting of a base sequence of SEQ ID NO:12 and a reverse primer consisting of a base sequence of SEQ ID NO:13,
- A primer pair 7a: a combination of a forward primer consisting of a base sequence of SEQ ID NO : 14 and a reverse primer consisting of a base sequence of SEQ ID NO:15

(Group B)
A primer pair in which one or two bases have been added, deleted or replaced in part of the sequence of one or both primers of each primer pair in (group A) above without impairing their function as primers, and

(Group C)
A primer pair consisting of a complementary sequence corresponding to the base sequence of one or both primers of each primer pair in (Group A) or (Group B) above.

[6] The bacterial identification method as according to any one of [1] to [5] above, characterized in that in the step (3), Tm value of the generated double-stranded DNA is measured, and the obtained Tm value is compared with the Tm value of the double-stranded DNA including the sequence for identification of the bacterium of interest that has been measured beforehand, or data obtained secondarily from them, to identify the bacterium in the sample.

[7] The bacterial identification method according to any one of [1] to [6] above, characterized in that, after the step (1), the reaction mixture is directly alone or diluted and used in the step (2).

[8] A kit for identifying bacteria in a sample by PCR using as a template a cDNA contained in a reaction mixture by reverse transcription reaction of an RNA extracted from bacteria in the sample, characterized in that the kit comprising (a) to (c) below:

(a) a primer for reverse transcription to prepare a cDNA containing a base sequence for identification of the bacterium of interest for preparation of the first reaction system for reverse transcription reaction,
(b) a primer pair for synthesis of a double-stranded DNA containing the base sequence for the identification of the bacteria of interest, for the preparation of a second reaction system for the PCR, and
(c) an enzyme with RNA-dependent DNA polymerase activity,

wherein, the second reaction system comprises the reaction mixture by the reverse transfer reaction, wherein the amount of primer for reverse transcription is adjusted such that the concentration of the primer for reverse transcription when fed through the reaction mixture to the second reaction system is 0.08nM or more and 20nM or less.

[9] The kit according to [8] above, characterized in that further comprising an instruction manual describing the reverse transcription reaction and the PCR method.

[10] The kit according to [9] above, characterized in that the instruction manual describes the procedure so that the concentration of the primer for reverse transcription in the second reaction system is not less than 0.08 nM and not more than 20 nM.

[11] The kit according to any one of [8] to [10] above, characterized in that the basic sequence for identification of the bacterium of interest is a basic sequence contained in a 16S rDNA of the bacterium and the RNA includes a 16S rRNA of the bacterium in the sample.

[12] The kit according to [11] above, characterized in that the primer for reverse transcription consist of a base sequence of SEQ ID NO : 1.

[13] The kit according to [11] to [12] above, characterized in that the primer pair is at least one primer pair selected from the group consisting of the following (Group A) to (Group C):

(Group A)

- A primer pair 1a: a combination of a forward primer consisting of a base sequence of SEQ ID NO : 2 and a reverse primer consisting of a base sequence of SEQ ID NO : 3,
- A primer pair 2a: a combination of a forward primer consisting of a base sequence of SEQ ID NO : 4 and a reverse primer consisting of a base sequence of SEQ ID NO : 5,
- A primer pair 3a: a combination of a forward primer consisting of a base sequence of SEQ ID NO : 6 and a reverse primer consisting of a base sequence of SEQ ID NO : 7,
- A primer pair 4a: a combination of a forward primer consisting of a base sequence of SEQ ID NO : 8 and a reverse primer consisting of a base sequence of SEQ ID NO : 9,
- A primer pair 5a: a combination of a forward primer consisting of a base sequence of SEQ ID NO : 10 and a reverse primer consisting of a base sequence of SEQ ID NO : 11, ▪ A primer Pair 6a: a combination of a forward primer consisting of a base sequence of SEQ ID NO:12 and a reverse primer consisting of a base sequence of SEQ ID NO:13,
- A primer pair 7a: a combination of a forward primer consisting of a base sequence of SEQ ID NO : 14 and a reverse primer consisting of a base sequence of SEQ ID NO : 15

(Group B)
A primer pair in which one or two bases have been added, deleted or replaced in part of the sequence of one or both primers of each primer pair in (group A) above without impairing their function as primers, and

(Group C)
A primer pair consisting of a complementary sequence corresponding to the base sequence of one or both primers of each primer pair in (Group A) or (Group B) above.

Effect of the invention

According to the present invention, detection and identification of bacteria can be performed even in 1 PCR while maintaining detection sensitivity.
Therefore, it is possible to reduce the workload and automate the system.
By appropriately adjusting the concentration of the primer for reverse transfer in the second reaction system, the identification method of the present invention is capable of detecting and identifying bacteria with high sensitivity even for a sample containing a dilute bacterium having a bacterial count of about 100 CFU/sample.

BRIEF DESCRIPTION OF THE DRAWINGS

[Fig.1] It is the figure showing the result of agarose electrophoresis of the PCR product in Example 1

[Fig.2] It is the figure showing the result of agarose electrophoresis of the PCR product in Example 2
[Fig.3] It is the figure showing the result of agarose electrophoresis of the PCR product in Example 3

## DETAILED DESCRIPTION OF THE INVENTION

A bacterial identification method according to the present invention has the following steps:

(I) A step of obtaining a reaction mixture comprising a cDNA by reverse transcription reaction using a RNA extracted from bacteria in a sample.
(II) A step of performing PCR using the reaction mixture containing the cDNA and a primer pair for bacterial identification.
(III) A step of detecting the generation of a double-stranded DNA amplified by PCR with a primer pair for bacterial identification.

For reverse transcription reaction in step (I), a first reaction system is used, including a primer for reverse transcription to prepare a cDNA containing a base sequence for identification of a bacterium of interest, an RNA extracted from the bacterium in a sample, and an enzyme with RNA-dependent DNA polymerase activity.
The reaction mixture in the present invention means a mixture containing a reaction mixture obtained by performing a reverse transfer reaction in a first reaction system and an unreacted product remaining unreacted without participating in the reaction.
The Primers for reverse-transcription used in the first reaction system are consumed during the synthesis of cDNA, but are usually used in excessive quantities greater than those required for the synthesis of cDNA of interest. Therefore, the reaction mixture by the reverse transfer reaction by the first reaction system includes a synthesized cDNA as a reaction product and a primer for reverse transfer remaining as an unreacted product.
For PCR in step (II), a second reaction system is used, including the reaction mixture, a primer pair for synthesis of a double-stranded DNA containing the base sequence for identification of the bacterium of interest, and an enzyme with DNA-dependent DNA polymerase activity,
[0037]    As described above, the reaction mixture obtained in Step (I) contains a primer for reverse transfer which has not been consumed in the reverse transfer reaction, and high sensitivity detection can be performed by adjusting the amount of the primer for this reverse transfer in Step (II)
For this purpose, the concentration of the primer for reverse transfer upon preparation of the second reaction system is selected from 0.08nM to 20nM, more preferably from 0.4nM to 20nM, even more preferably from 0.8nM to 20nM, and even more preferably from 2nM to 20nM.
If the amount of primer brought into the second reaction system for reverse transcription is high, the PCR amplification product may contain an undesired amplification product, resulting in a decrease in identification accuracy due to an increase in the background in bacterial identification. Therefore, the concentration of the primer for reverse transfer in the second reaction system is set to the above range.
The Steps (I) and (II) can be carried out in sequence in different reaction vessels or in the same reaction vessel.
The kit for bacterial identification according to the present invention has the following constituents (a) to (c):

(a) a primer for reverse transcription to prepare a cDNA containing a base sequence for identification of the bacterium of interest for preparation of the first reaction system for reverse transcription reaction,
(b) a primer pair for synthesis of a double-stranded DNA containing the base sequence for the identification of the bacteria of interest, for the preparation of a second reaction system for the PCR, and
(c) an enzyme with RNA-dependent DNA polymerase activity,

In addition to the kit for bacterial identification according to the present invention, if desired, it may have (d) an enzyme with DNA-dependent DNA polymerase activity for the preparation of a second reaction system.
The amount of the primer for reverse transfer contained in the kit is adjusted so that the concentration when supplied to the second reaction system via the reaction mixture obtained by the reverse transfer reaction is 0.08nM to 20nM, more preferably 0.4nM to 20nM, still more preferably 0.8nM to 20nM, and still more preferably 2nM to 20nM.
Noted that the bacterial identification method and the kit for the same according to the present invention can be also used for a detection method for detecting the presence or absence of a detection target bacterium in a sample.
Hereinafter, a preferred embodiment of a bacterial identification method and a kit for the same according to the present invention will be described.

<RNA extraction>

As an RNA extraction method, an alkaline dissolution method, a boiling method, a phenol extraction, and the like are known, and a dedicated RNA extraction kit is also sold from each manufacturer

A method for extracting RNA from bacteria in a sample in the present invention is not particularly limited, and a known method can be used.

Since the most appropriate method differs depending on the sample, it is desirable to select a method appropriate for the target sample.

Note that High Pure RNA Isolation Kit manufactured by Roche Co., Ltd., which is used in the present embodiment, is an example of an RNA-extracting method which can be suitably used.

<Reverse transcription reaction>

The RNA obtained from bacteria in the sample needs to be made into cDNA by a reverse transcription reaction. The reverse transcription reaction method in the present invention is not particularly limited and known methods can be used. Promega's M-MLV Reverse Transcriptase, RNase (H-), Point Mutant, which is used in the examples described below, is an example of a suitably usable reverse transcription reaction method.

The amount of RNA and RNA-dependent DNA polymerase to be added to the first reaction system is not particularly limited, and an amount capable of synthesizing the target cDNA may be selected.

<Primer for reverse transcription>

The Primer for reverse transcription are used to synthesize a cDNA containing the sequence used for bacterial identification by reverse transcription. The Primer for reverse-transcription are not particularly limited if they are capable of synthesizing cDNA (fragments) containing sequences complementary to those required for bacterial identification in RNA extracted from bacteria in a sample. Known or newly prepared primers capable of synthesizing DNA containing a base sequence for bacterial identification can be used. Commercially available primers are also available.

As a base sequence for bacterial identification, many base sequences are known in 16S rDNA, and it is preferable to use 16S rRNA corresponding to 16S rDNA as RNA for bacterial identification.

If the bacterial 16S rRNA is targeted, a known primer or a newly prepared primer that is complementary to the starting position of the synthesis of a cDNA containing a region of the 16S rRNA that can be exploited for bacterial identification can be used as a primer for reverse transcription. Commercially available primers are also available.

It is preferable to select primers for reverse transcription so that cDNA synthesized by the primer for reverse transcription has a region capable of amplification by the primer pair for bacterial identification as described below.

Examples of particularly suitable primers for reverse transcription include primers consisting of the base sequence of SEQ ID NO : 1 described below.

SEQ ID NO1:AGACCCGGGA ACGTATTC

The amount of reverse transcription primer to be added to the first reaction system is adjusted so that the amount of reverse transcription primer added to the first reaction system is necessary for the target reverse transcription reaction and the concentration of the reverse transcription primer in the second reaction system to which the reaction mixture of the reverse transcription reaction using the first reaction system is added is less than 20 nM.

In addition, the lower limit of the concentration of the primer for reverse transcription in the second reaction system is such a concentration that cDNA can be synthesized from the RNA to such an extent that the bacteria present in the sample can be detected and identified. From this point of view, the concentration of the primer for reverse transfer of the second reaction system may preferably be adjusted to be in the range of 0.08nM to 20nM, more preferably 0.4nM to 20nM, still more preferably 0.8 nM to 20nM, and even more preferably 2nM to 20nM.

The following methods can be used to adjust the amount of primers for reverse transcription:

(A) When the reaction mixture obtained by reverse transcription in the first reaction system is directly used alone for PCR in the second reaction system Calculate the amount of the reaction mixture obtained in the first reaction system and the carrying-in concentration of the primer for reverse transfer by the reaction mixture relative to the total amount of the amount of the second reaction system containing this reaction mixture. Note that the amount of primers for reverse transcription consumed in the reverse transcription reaction is not considered in the calculation of this concentration. Therefore, the amount of the reverse transfer primer used in the calculation of this concentration is determined based on the amount of the reverse transfer primer added to the first reaction system.

The concentration of the primer for reverse transfer to be added to the first reaction system is set so that the concentration of the primer for reverse transfer brought into the second reaction system thus calculated falls within the range described above.

Based on the concentration of the primer for reverse transfer of the first reaction system set as described above, it is preferable to set the composition of the first reaction system and the conditions for the reverse transfer reaction so that the target reverse transfer reaction can be performed.

For example, when the amount of the second reaction system is 10 times the amount of the reaction mixture obtained from the first reaction system, the concentration of the primer for reverse transfer to be added to the first reaction system may be set to 10 times the concentration of the primer for reverse transfer contained in the second reaction system. If the concentration of primer for reverse transfer in the second reaction system is set to 0.08nM to 20nM as the preferred range described above, the concentration of primer for reverse transfer in the first reaction system is set to the range of 0.8nM to 200nM.

By keeping the primer concentration for reverse rotation transcription sufficiently in excess of the amount required for reverse transcription, this amount can be calculated approximately without considering the amount of primer consumed in the reverse transcription reaction in calculating the amount of the primer for reverse transcription taken into the second reaction system described above.

Further, the present invention is characterized in that an upper limit value of an amount of a primer for reverse transfer brought into a second reaction system is set as a threshold value for obtaining an effect of the present invention. By setting the concentration of primers for reverse rotation transcription that are added to the first reaction system without considering the amount of primers consumed in the reverse transcription reaction as described above, the amount of primers for reverse transcription that are brought into the second reaction system after subtracting the amount consumed in the reverse transcription reaction is automatically below the threshold mentioned above.

In the above adjustment method (A), the reaction mixture obtained by the reverse transfer reaction is used as it is alone, that is, directly in the preparation of the second reaction system without any addition and without any separation from the reaction mixture.

(B) When the reaction mixture obtained by reverse transcription in the first reaction system is diluted and used for PCR in the second reaction system

When the concentration of the primer for reverse transfer necessary for carrying out the target reverse transfer reaction is used, when the reaction mixture for reverse transfer reaction is used in the preparation of the second reaction solution according to the above-described adjustment method (A), when the concentration of the primer for reverse transfer exceeds 20nM in the second reaction system, the reaction organism obtained from the first reaction system is diluted so as to be the concentration of the primer for reverse transfer in the above-described second reaction system. The dilution rate in this dilution can be set based on the concentration of the primer for reverse transfer in the first reaction system, the amount of the reaction mixture obtained from the first reaction system, and the amount of the second reaction system.

Also in the calculation of the concentration of the primer for reverse transfer in this adjustment method (B), the amount of the primer consumed in the reverse transfer reaction in the first reaction system can be ignored.

In addition, for dilution of the reaction mixture, a liquid medium such as a buffer solution for dilution of a primer solution or a liquid medium such as a buffer solution utilized for preparation of a second reaction system for PCR can be utilized.

<Primers for PCR>

As a primer pair for PCR, that is, a combination of a forward primer and a reverse primer, there is no limitation as long as it can amplify a double-stranded DNA containing a base sequence for bacterial identification. The primer pairs can be selected according to the bacterial species being identified.

When targeting a bacterial 16S rDNA, a primer pair capable of amplifying a base sequence for bacterial identification in the 16S rDNA can be used for bacterial identification. A variety of bacterial identification sequences are known in the 16S rDNA, and primer pairs can use primer pairs capable of amplifying double-stranded DNA containing a base sequence for known bacterial identification.

As will be described later, when a plurality of DNA fragments are amplified from a cDNA by PCR and a bacterium is identified based on the Tm value of the DNA fragments, it is preferable to use a primer pair in which a double-stranded DNA containing each of the variable regions (see S. Chakravorty, et al., Journal of Microbiological Methods 2007, 69, 330-339) present on the 16S rDNA is amplified.

The Primer pair for PCR can use one or multiple combinations.

When multiple primer sets are used, it is preferable to use each of the four to seven primer pairs individually in order to increase the number of identified species or to improve the accuracy of identification.

Preferred primer pairs may include at least one primer pair selected from the group consisting of (Group A) to (Group C) below.

(Group A)

- A primer pair 1a: a combination of a forward primer consisting of a base sequence of SEQ ID NO : 2 and a reverse primer consisting of a base sequence of SEQ ID NO : 3,
- A primer pair 2a: a combination of a forward primer consisting of a base sequence of SEQ ID NO : 4 and a reverse primer consisting of a base sequence of SEQ ID NO : 5,
- A primer pair 3a: a combination of a forward primer consisting of a base sequence of SEQ ID NO : 6 and a reverse primer consisting of a base sequence of SEQ ID NO : 7,
- A primer pair 4a: a combination of a forward primer consisting of a base sequence of SEQ ID NO : 8 and a reverse primer consisting of a base sequence of SEQ ID NO : 9,
- A primer pair 5a: a combination of a forward primer consisting of a base sequence of SEQ ID NO : 10 and a reverse primer consisting of a base sequence of SEQ ID NO : 11,
- A primer Pair 6a: a combination of a forward primer consisting of a base sequence of SEQ ID NO:12 and a reverse primer consisting of a base sequence of SEQ ID NO : 13,
- A primer pair 7a: a combination of a forward primer consisting of a base sequence of SEQ ID NO : 14 and a reverse primer consisting of a base sequence of SEQ ID NO : 15

(Group B)

A primer pair in which one or two bases have been added, deleted or replaced in part of the sequence of one or both primers of each primer pair in (group A) above without impairing their function as primers, and

(Group C)

A primer pair consisting of a complementary sequence corresponding to the base sequence of one or both primers of each primer pair in (Group A) or (Group B) above.
Specific examples of the PCR primers described above include primer pairs described in Groups 1 to 7 below. It is more preferred to use 4-7 primer pairs of each of the 7 primer pairs selected from each of these 7 groups individually.

(1) A primer pair group 1:

(1a) A combination of a forward primer consisting of a base sequence of SEQ ID NO : 2 and a reverse primer consisting of a reverse primer consisting of a base sequence of SEQ ID NO : 3
(1b-1) A primer pair consisting of a combination of a modified forward primer consisting of a modified base sequence of SEQ ID NO : 2 and a reverse primer consisting of SEQ ID NO : 3
(1b-2) A primer pair consisting of a combination of a forward primer consisting of SEQ ID NO : 2 and a modified reverse primer consisting of a modified base sequence of SEQ ID NO : 3
(1b-3) A primer pair consisting of a combination of a modified forward primer consisting of a modified base sequence of SEQ ID NO : 2 and a modified reverse primer consisting of a modified base sequence of SEQ ID NO : 3
(1c-1) A primer pair consisting of a forward primer consisting of a complementary sequence of SEQ ID NO : 2 and a reverse primer consisting of a complementary sequence of SEQ ID NO : 3
(1c-2) A primer pair consisting of a combination of a modified forward primer consisting of a complementary sequence of a modified base sequence of SEQ ID NO : 2 and a reverse primer consisting of a complementary sequence of SEQ ID NO : 3
(1c-3) A primer pair consisting of a combination of a forward primer consisting of a complementary sequence of SEQ ID NO : 2 and a modified reverse primer consisting of a complementary sequence of a modified base sequence of SEQ ID NO : 3
(1c-4) A primer pair consisting of a combination of a modified forward primer consisting of a complementary sequence of a modified base sequence of SEQ ID NO : 2 and a modified reverse primer consisting of a complementary sequence of a modified base sequence of SEQ ID NO : 3

(2) A primer pair group 2:

(2a) In combination with a forward primer consisting of sequence number 4 sequences and a reverse primer consisting of sequence number 5 sequences,

(2b-1) A primer pair consisting of a combination of a modified forward primer consisting of a modified base sequence of SEQ ID NO : 4 and a reverse primer consisting of SEQ ID NO : 5

(2b-2) A primer pair consisting of a combination of a forward primer consisting of SEQ ID NO : 4 and a modified reverse primer consisting of a modified base sequence of SEQ ID NO : 5

(2b-3) A primer pair consisting of a combination of a modified forward primer consisting of a modified base sequence of SEQ ID NO : 4 and a modified reverse primer consisting of a modified base sequence of SEQ ID NO : 5

(2c-1) A primer pair consisting of a forward primer consisting of a complementary sequence of SEQ ID NO : 4 and a reverse primer consisting of a complementary sequence of SEQ ID NO : 5

(2c-2) A primer pair consisting of a combination of a modified forward primer consisting of a complementary sequence of a modified base sequence of SEQ ID NO : 4 and a reverse primer consisting of a complementary sequence of SEQ ID NO : 5

(2c-3) A primer pair consisting of a combination of a forward primer consisting of a complementary sequence of SEQ ID NO : 4 and a modified reverse primer consisting of a complementary sequence of a modified base sequence of SEQ ID NO : 5

(2c-4) A primer pair consisting of a combination of a modified forward primer consisting of a complementary sequence of a modified base sequence of SEQ ID NO : 4 and a modified reverse primer consisting of a complementary sequence of a modified base sequence of SEQ ID NO : 5

(3) Primer pairs, Group 3:

(3a) In combination with a forward primer consisting of sequence number 6 sequences and a reverse primer consisting of sequence number 7 sequences,

(3b-1) A primer pair consisting of a combination of a modified forward primer consisting of a modified base sequence of SEQ ID NO : 6 and a reverse primer consisting of SEQ ID NO : 7

(3b-2) A primer pair consisting of a combination of a forward primer consisting of SEQ ID NO : 6 and a modified reverse primer consisting of a modified base sequence of SEQ ID NO : 7

(3b-3) A primer pair consisting of a combination of a modified forward primer consisting of a modified base sequence of SEQ ID NO : 6 and a modified reverse primer consisting of a modified base sequence of SEQ ID NO : 7

(3c-1) A primer pair consisting of a forward primer consisting of a complementary sequence of SEQ ID NO : 6 and a reverse primer consisting of a complementary sequence of SEQ ID NO : 7

(3c-2) A primer pair consisting of a combination of a modified forward primer consisting of a complementary sequence of a modified base sequence of SEQ ID NO : 6 and a reverse primer consisting of a complementary sequence of SEQ ID NO : 7

(3c-3) A primer pair consisting of a combination of a forward primer consisting of a complementary sequence of SEQ ID NO : 6 and a modified reverse primer consisting of a complementary sequence of a modified base sequence of SEQ ID NO : 7

(3c-4) A primer pair consisting of a combination of a modified forward primer consisting of a complementary sequence of a modified base sequence of SEQ ID NO : 6 and a modified reverse primer consisting of a complementary sequence of a modified base sequence of SEQ ID NO : 7

(4) Primer pair group 4:

(4a) In combination with a forward primer consisting of sequence number 8 sequences and a reverse primer consisting of sequence number 9 sequences,

(4b-1) A primer pair consisting of a combination of a modified forward primer consisting of a modified base sequence of SEQ ID NO : 8 and a reverse primer consisting of SEQ ID NO : 9

(4b-2) A primer pair consisting of a combination of a forward primer consisting of SEQ ID NO : 8 and a modified reverse primer consisting of a modified base sequence of SEQ ID NO : 9

(4b-3) A primer pair consisting of a combination of a modified forward primer consisting of a modified base sequence of SEQ ID NO : 8 and a modified reverse primer consisting of a modified base sequence of SEQ ID NO : 9

(4c-1) A primer pair consisting of a forward primer consisting of a complementary sequence of SEQ ID NO : 8 and a reverse primer consisting of a complementary sequence of SEQ ID NO : 9

(4c-2) A primer pair consisting of a combination of a modified forward primer consisting of a complementary sequence of a modified base sequence of SEQ ID NO : 8 and a reverse primer consisting of a complementary sequence of SEQ ID NO : 9

(4c-3) A primer pair consisting of a combination of a forward primer consisting of a complementary sequence of SEQ ID NO : 8 and a modified reverse primer consisting of a complementary sequence of a modified base sequence of SEQ ID NO : 9 (4c-4) A primer pair consisting of a combination of a modified forward primer consisting of a complementary sequence of a modified base sequence of SEQ ID NO : 8 and a modified reverse primer consisting of a complementary sequence of a modified base sequence of SEQ ID NO : 9

(5) A primer pair group 5:

(5a) A combination of a forward primer consisting of a base sequence of SEQ ID NO:10 and a reverse primer consisting of a base sequence of SEQ ID NO:11,

(5b-1) A primer pair consisting of a combination of a modified forward primer consisting of a modified base sequence of SEQ ID NO : 10 and a reverse primer consisting of SEQ ID NO : 11

(5b-2) A primer pair consisting of a combination of a forward primer consisting of SEQ ID NO : 10 and a modified reverse primer consisting of a modified base sequence of SEQ ID NO : 11

(5b-3) A primer pair consisting of a combination of a modified forward primer consisting of a modified base sequence of SEQ ID NO : 10 and a modified reverse primer consisting of a modified base sequence of SEQ ID NO : 11

(5c-1) A primer pair consisting of a forward primer consisting of a complementary sequence of SEQ ID NO : 10 and a reverse primer consisting of a complementary sequence of SEQ ID NO : 11

(5c-2) A primer pair consisting of a combination of a modified forward primer consisting of a complementary sequence of a modified base sequence of SEQ ID NO : 10 and a reverse primer consisting of a complementary sequence of SEQ ID NO : 11

(5c-3) A primer pair consisting of a combination of a forward primer consisting of a complementary sequence of SEQ ID NO : 10 and a modified reverse primer consisting of a complementary sequence of a modified base sequence of SEQ ID NO : 11

(5c-4) A primer pair consisting of a combination of a modified forward primer consisting of a complementary sequence of a modified base sequence of SEQ ID NO : 10 and a modified reverse primer consisting of a complementary sequence of a modified base sequence of SEQ ID NO : 11

(6) Primer pair group 6:

(6a) A combination of a forward primer consisting of a base sequence of SEQ ID NO:12 and a reverse primer consisting of a base sequence of SEQ ID NO:13,

(6b-1) A primer pair consisting of a combination of a modified forward primer consisting of a modified base sequence of SEQ ID NO : 12 and a reverse primer consisting of SEQ ID NO : 13

(6b-2) A primer pair consisting of a combination of a forward primer consisting of SEQ ID NO : 12 and a modified reverse primer consisting of a modified base sequence of SEQ ID NO : 13

(6b-3) A primer pair consisting of a combination of a modified forward primer consisting of a modified base sequence of SEQ ID NO : 12 and a modified reverse primer consisting of a modified base sequence of SEQ ID NO : 13

(6c-1) A primer pair consisting of a forward primer consisting of a complementary sequence of SEQ ID NO : 12 and a reverse primer consisting of a complementary sequence of SEQ ID NO : 13

(6c-2) A primer pair consisting of a combination of a modified forward primer consisting of a complementary sequence of a modified base sequence of SEQ ID NO : 12 and a reverse primer consisting of a complementary sequence of SEQ ID NO : 13

(6c-3) A primer pair consisting of a combination of a forward primer consisting of a complementary sequence of SEQ ID NO : 12 and a modified reverse primer consisting of a complementary sequence of a modified base sequence of SEQ ID NO : 13

(6c-4) A primer pair consisting of a combination of a modified forward primer consisting of a complementary sequence of a modified nucleotide sequence of SEQ ID NO : 12 and a modified reverse primer consisting of a complementary sequence of a modified nucleotide sequence of SEQ ID NO : 13

(7) Primer pair group 7:

(7a) Combination with a forward primer consisting of a base sequence of SEQ ID NO:14 and a reverse primer consisting of a base sequence of SEQ ID NO: 15,

(7b-1) A primer pair consisting of a combination of a modified forward primer consisting of a modified base sequence of SEQ ID NO : 14 and a reverse primer consisting of SEQ ID NO : 15

(7b-2) A primer pair consisting of a combination of a forward primer consisting of SEQ ID NO : 14 and a modified reverse primer consisting of a modified base sequence of SEQ ID NO : 15

(7b-3) A primer pair consisting of a combination of a modified forward primer consisting of a modified base sequence of SEQ ID NO : 14 and a modified reverse primer consisting of a modified base sequence of SEQ ID NO : 15

(7c-1) A primer pair consisting of a forward primer consisting of a complementary sequence of SEQ ID NO : 14 and a reverse primer consisting of a complementary sequence of SEQ ID NO : 15

(7c-2) A primer pair consisting of a combination of a modified forward primer consisting of a complementary sequence of a modified base sequence of SEQ ID NO : 14 and a reverse primer consisting of a complementary sequence of SEQ ID NO : 15

(7c-3) A primer pair consisting of a combination of a forward primer consisting of a complementary sequence of SEQ ID NO : 14 and a modified reverse primer consisting of a complementary sequence of a modified base sequence of SEQ ID NO : 15

(7c-4) A primer pair consisting of a combination of a modified forward primer consisting of a complementary sequence of a modified nucleotide sequence of SEQ ID NO : 14 and a modified reverse primer consisting of a complementary sequence of a modified nucleotide sequence of SEQ ID NO : 15

A base sequences of SEQ ID NOs : 2 to 15 are shown below.

SEQ ID NO2: GCAGGCTTAA CACATGCAAG TCG
SEQ ID NO3: CGTAGGAGTC TGGACCGT
SEQ ID NO4: GTCCAGACTC CTACGGGAG
SEQ ID NO5: CCTACGTATT ACCGCGG
SEQ ID NO6: AGCAGCCGCG GTAATA
SEQ ID NO7: GGACTACCAG GGTATCTAAT CCT
SEQ ID NO8: AACAGGATTA GATACCCTGG TAG
SEQ ID NO9: AATTAAACCA CATGCTCCAC C
SEQ ID NO10: TGGTTTAATT CGATGCAACG C
SEQ ID NO11: GAGCTGACGA CAGCCAT
SEQ ID NO12: GTTAAGTCCC GCAACGAG
SEQ ID NO13 : CCATTGTAGC ACGTGTGTAG CC
SEQ ID NO14: GGCTACACAC GTGCTACAAT GG
SEQ ID NO15: AGACCCGGGA ACGTATTC

<Reagents for PCR>

Reagents other than primer pairs used for PCR analysis in the present invention may be used by known combinations.

Reagents required for the determination include enzymes for PCR, pH buffers, dNTP, $Mg^{2+}$ sources, sterilized water purified by filtration treatment, and the like.

In addition, in real-time PCR analysis, fluorescent dyes is required in addition to the above.

<Enzymes for PCR>

The enzyme for PCR used in the present invention is a DNA-dependent DNA polymerase, which can be used in a variety of commercially available samples, but DNA-dependent thermostable DNA polymerase with minimal DNA contamination from bacteria causing false positives is preferred in studies involving particularly negative samples.

Specific examples thereof include, but are not limited to, those produced in eukaryotes, those subjected to advanced purification treatment, or those treated with EMAs (ethidium monoazide) or PMAs (propidium monoazide), which are selective membrane permeable dyes.

<PCR analysis>

The cDNA and primer pairs as reaction products obtained by the reverse transcription reaction are used in PCR methods. A variety of PCR methods can be used as PCR methods for amplification of the double-stranded DNA containing the base sequence for identification of the bacteria. A preferred analytical method is real-time PCR, more preferably a combination of real-time PCR and melting curve analysis for Tm value measurement.

The conditions for temperature cycling in PCR are not specifically limited, but one example is the conditions implemented in the following Examples; "95° C., 5 minutes → 94° C., 10 seconds ▪ 60° C., 20 econds ▪ 72° C., 10 seconds ▪ 85° C., and 2 seconds for 40 cycles → 95° C., 10 seconds. "

In real-time PCR, after the DNA amplification step is completed, the Tm value of the amplified DNA can be measured by melting curve analysis.

These measurements are possible with many models of real-time PCR equipment and can be performed in accordance with the method of use of the equipment.

<Identification method>

To identify bacteria in a sample, the Tm-value of a double-stranded DNA fragment contained in an amplification product obtained from a reaction mixture comprising a cDNA using a primer pair according to the present invention can be utilized.

Preferred identification methods include the following.

First, double-stranded DNA fragments and their Tm values are previously acquired using 16S rRNAs or 16S rDNAs of multiple species, preferably a number of bacterial species that may be included in the sample, by the methods described in the present invention, or by methods utilizing a portion of the methods described in the invention, and their Tm values (measurements themselves) or secondary data from the measurement Tm values are obtained and stored. For example, the relative value of the Tm value, which will be described later, is stored as comparison data or as a database. This comparison data is a pre-acquired "relative value of Tm value" of a double-stranded DNA fragment obtained from a known bacterium. The database for this comparison data also stores data that have been comprehensively retrieved from the above comparative data for double-stranded DNA fragments from multiple bacteria.

Next, the relative values of the Tm or "Tm values" obtained from bacteria in the sample can be compared with this conserved comparison data or database to identify bacteria of unknown species in the sample.

As an algorithm for identification, not only the combination of Tm values as described above, but also the combination of differences between each Tm value can be used to identify, which adds a step to minimize the influence of measurement error, for example, measurement error on a trial-by-trial basis.

As a method for correcting the measurement error of each trial of the instrument described above, the average value of the combination of "Tm values" obtained for each of the multiple primer pairs can be calculated, and the "combination of relative values" of each Tm value from the average value can be used. For this relative value, the difference between each Tm value from the mean can be used, and this relative value is the relative value of the previously described "Tm value". In other words, it is a method for identifying the arrangement of combinations of Tm values as "shapes". The "shape" which shows the arrangement of the combination of Tm values in two dimensions is not affected by the measurement error.

For example, a combination of Tm-values specific to the detected bacteria (n (n is an integer between 4 and 7)) is taken as T1db~Tndb (db is database), and the relative values from that mean are taken as d1db~dndb, respectively. Similarly, a combination of Tm-values of unknown detected object organisms obtained from a sample (n (n is an integer between 4 and 7)) is used as a T1ref~Tnref (ref is reference), and the relative values from that mean are d1ref~dnref, respectively. Compared to database, we then utilize "approximate combinations of relative values = closer "shapes" of combinations of Tm values" as identification algorithms.

Specific computational methods include, but are not limited to, calculating the two-point distance in Euclidean space (Formula 1).

[Formula 1]

$$\mathrm{Dist.} = \sqrt{[(D1_{db} - D1_{ref})^2 + (D2_{db} - D2_{ref})^2 + \cdots (Dn_{db} - Dn_{ref})^2]} \cdots (\text{Formula } 1)$$

If the calculation method is based on Formula 1, the bacterial species whose Dist. value is closest to 0 is identified as the species to be detected. Note that the acceptable Dist. values are 0 to 0.37 and preferably 0 to 0.30, depending on the number of temperature-controlled specks and primers used for the PCR equipment, depending on the measurement error of the PCR equipment used.

These algorithms can be used as database identification software on computers.

Identification of bacterial species using the relative values of the above Tm values, that is, identification of bacterial species by the Tm mapping method, may be performed according to, for example, the method disclosed in Patent Document 2, paragraph [0237] of WO2010/082640, or paragraphs [0111] to [0116] of WO2015/053293, or by appropriately modifying the methods disclosed therein.

In one example of the identification of bacterial species by the Tm mapping method, identification data on a combination of Tm values of multiple amplification products amplified by multiple specific primer pairs obtained from a bacterium in which a bacterial species is known or a combination of differences between multiple Tm values are utilized.

Combinations of the Tm values of amplification products obtained by the same multiple primer pairs from unknown cell samples collected from the sample, or a combination of differences between the Tm values, are collated with the identification database to determine the consistency and identify unknown bacteria in the sample.

<Kits for bacterial identification>

The kit for bacterial identification according to the present invention has each of the following components (a) to (c) described above.

(a) a primer for reverse transcription to prepare a cDNA containing a base sequence for identification of the bacterium of interest for preparation of the first reaction system for reverse transcription reaction,
(b) a primer pair for synthesis of a double-stranded DNA containing the base sequence for the identification of the bacteria of interest, for the preparation of a second reaction system for the PCR, and
(c) an enzyme with RNA-dependent DNA polymerase activity,

In addition to these constituents, it may have the following (d)
(d) An enzyme with DNA-dependent DNA polymerase activity for the preparation of a second reaction system.

In addition, an instruction manual describing the procedures for the reverse transcription reaction and PCR, e.g., steps (I)-(III) listed above, may be attached to the kit.

Additionally, the kit may be accompanied by an instruction manual describing the reverse transcription reaction and the PCR method, for examples, the procedures for steps (I)-(III) previously listed.

In addition, it is preferable that the instruction manual describe, for example, the preparation method (A) and (B) mentioned above such that the concentration of the primer for reverse transcription contained in the second reaction system is 20nM or less, preferably 0.08nM to 20nM, more preferably 0.4nM to 20nM, still more preferably 0.8 nM to 20nM, and still more preferably 2nM to 20nM.

The instruction manual may include an explanation of the fact that the reaction using the first reaction system and the reaction using the second reaction system can be carried out in this order in different reaction vessels or in the same reaction vessel.

The kit for bacterial identification may have at least one of the reagents listed in the section "Reagents for PCR" above, for example, enzymes for PCR, pH buffers, dNTP, $Mg^{2+}$ sources, sterilized water purified by filtration treatment or the like, and fluorescent dyes for real-time PCR analysis.

In addition, if the Tm values described above are used to identify bacterial species, Tm values obtained from known bacterial species or media containing comparative data or databases may be added to the kit.

These stored data may also be provided to the user via the Internet, and the instruction manual may include an explanation of this point and an explanation of the database type identification software available on the computer described above.

The kit may also further comprise a positive control for identification, a negative control.

<Identifiable bacterial species>

Identifiable microorganisms are mechanistically detectable and bacterial species can be identified if they fall into the class Bacteria.

Specific bacterial species include, but are not limited to, Achromobacter denitrificans, Achromobacter xylosoxidans, Acinetobacter baumannii, Acinetobacter calcoaceticus, Actinomyces israelii, Aerococcus christensenii, Aeromonas hydrophila, Aeromonas sobria, Aggregatibacter actinomycetemcomitans, Alcaligenes faecalis, Alistipes onderdonkii, Anaerococcus vaginalis, Anaeroglobus geminatus, Arcanobacterium haemolyticum, Arcanobacterium pyogenes, Arthrobacter cumminsii, Atopobium vaginae, Bacillus anthracis, Bacillus cereus, Bacillus coagulans, Bacillus licheniformis, Bacillus megaterium, Bacillus pumilus, Bacillus sphaericus, Bacillus subtilis, Bacteroides dorei, Bacteroides finegoldii, Bacteroides fragilis, Bacteroides nordii, Bacteroides salyersiae, Bacteroides thetaiotaomicron, Bacteroides uniformis, Bacteroides vulgatus, Bartonella henselae, Bartonella quintana, Bifidobacterium bifidum, Bifidobacterium breve, Bilophila wadsworthia, Bordetella pertussis, Borrelia burgdorferi, Borrelia recurrentis, Brevibacillus laterosporus, Brucella abortus, Brucella melitensis, Brucella suis, Burkholderia cepacia, Burkholderia mallei, Burkholderia pseudomallei, Campylobacter coli, Campylobacter curvus, Campylobacter jejuni, Campylobacter rectus, Capnocytophaga gingivalis, Capnocytophaga granulosa, Capnocytophaga haemolytica, Capnocytophaga sputigena, Cardiobacterium hominis, Chryseobacterium meningosepticum, Citrobacter amalonaticus, Citrobacter freundii, Citrobacter koseri, Clostridium butyricum, Clostridium difficile, Clostridium histolyticum, Clostridium hylemonae, Clostridium paraputrificum, Clostridium perfringens, Clostridium septicum, Clostridium sporogenes, Clostridium subterminale, Clostridium tertium, Clostridium tetani, Corynebacterium amycolatum, Corynebacterium confusum, Corynebacterium diphtheriae, Corynebacterium glucuronolyticum, Corynebacterium jeikeium, Corynebacterium kroppenstedtii, Corynebacterium macginleyi, Corynebacterium minutissimum, Corynebacterium pseudodiphtheriticum, Corynebacterium pseudotuberculosis, Corynebacterium riegelii, Corynebacterium tuberculostearicum, Corynebacterium ulcerans, Corynebacterium xerosis, Edwardsiellatarda, Eggerthella lenta, Eikenella corrodens, Elizabethkingia meningoseptica, Empedobacter brevis, Enterobacter aerogenes, Enterobacter aerogenes, Enterobacter cloacae, Enterobacter sakazakii, Enterococcus avium, Enterococcus bovis, Enterococcus casseliflavus, Enterococcus cecorum, Enterococcus dispar, Enterococcus durans, Enterococcus faecium, Enterococcus flavescens, Enterococcus gallinarum, Enterococcus gilvus, Enterococcus hirae, Enterococcus italicus, Enterococcus malodoratus, Enterococcus mundtii, Enterococcus pallens, Enterococcus pseudoavium, Enterococcus raffinosus, Enterococcus sanguinicola, Erysipelothrix rhusiopathiae, Escherichia albertii, Escherichia coli, Eubacterium lentum, Eubacterium limosum, Finegoldia magna, Francisella tularensis, Fusobacterium necrophorum, Fusobacterium nucleatum, Fusobacterium periodonticum, Fusobacterium varium, Gardnerella vaginalis, Gemella morbillorum, Geobacillus stearothermophilus, Granulicatella adiacens, Haemophilus ducreyi, Haemophilus influenzae, Haemophilus parainfluenzae, Hafnia alvei, Halomonas venusta, Helicobacter cinaedi, Helicobacter pylori, Kingella kingae, Klebsiella granulomatis, Klebsiella oxytoca, Klebsiella pneumoniae, Lactobacillus acidophilus, Lactobacillus crispatus, Lactobacillus delbrueckii, Lactobacillus jensenii, Lactococcus garvieae, Legionella pneumophila, Leptospira interrogans, Listeria monocytogenes, Micrococcus luteus, Moraxella catarrhalis, Morganella morganii, Mycoplasma genitalium, Mycoplasma hominis, Neisseria gonorrhoeae, Neisseria meningitidis, Nocardia cyriacigeorgica, Odoribacter splanchnicus, Pantoea agglomerans, Parabacteroides distasonis, Parvimonas micra, Pasteurella multocida, Pediococcus damnosus, Peptoniphilus asaccharolyticus, Peptoniphilus gorbachii, Peptostreptococcus anaerobius, Plesiomonas shigelloides, Porphyromonas asaccharolytica, Porphyromonas gingivalis, Prevotella bivia, Prevotella bivia, Prevotella corporis, Prevotella intermedia, Prevotella melaninogenica, Prevotella nigrescens, Prevotella timonensis, Prevotella veroralis, Propionibacterium acnes, Propionibacterium avidum, Propionibacterium granulosum, Proteus mirabilis, Proteus vulgaris, Providencia rettgeri, Providencia stuartii, Pseudomonas aeruginosa, Pseudomonas fluorescens, Pseudomonas putida, Rothia dentocariosa, Rothia mucilaginosa, Salmonella enterica, Serratia marcescens, Serratia plymuthica, Shigella boydii, Shigella dysenteriae, Shigella flexneri, Shigella sonnei, Spirillum minus, Staphylococcus aureus, Staphylococcus auricularis, Staphylococcus capitis, Staphylococcus caprae, Staphylococcus carnosus, Staphylococcus cohnii, Staphylococcus epidermidis, Staphylococcus haemolyticus, Staphylococcus hominis, Staphylococcus lugdunensis, Staphylococcus pasteuri, Staphylococcus pettenkoferi, Staphylococcus pulvereri, Staphylococcus saccharolyticus, Staphylococcus saprophyticus, Staphylococcus schleiferi, Staphylococcus simulans, Staphylococcus warneri, Staphylococcus xylosus, Stenotrophomonas maltophilia, Streptobacillus moniliformis, Streptococcus agalactiae, Streptococcus anginosus, Streptococcus bovis, Streptococcus canis, Streptococcus constellatus, Streptococcus dysgalactiae, Streptococcus equi, Streptococcus gallolyticus, Streptococcus gordonii, Streptococcus infantarius, Streptococcus iniae, Streptococcus intermedius, Streptococcus lutetiensis, Streptococcus mitis, Streptococcus mutans, Streptococcus oralis, Streptococcus pasteurianus, Streptococcus pneumoniae, Streptococcus porcinus, Streptococcus pyogenes, Streptococcus salivarius, Streptococcus salivarius, Streptococcus sanguinis, Streptococcus sobrinus, Streptococcus suis, Streptococcus vestibularis, Sutterella wadsworthensis, Treponema pallidum, Ureaplasma parvum, Vagococcus fluvialis, Veillonella atypica, Veillonella parvula, Vibrio alginolyticus, Vibrio cholerae, Vibrio fluvialis, Vibrio parahaemolyticus, Vibrio vulnificus, Yersinia enterocolitica, Yersinia pestis, Yersinia pseudotuberculosis.

Examples

(Manufacturing Example 1)

The DNA polymerase used in the present invention was produced by the method described in paragraphs 195 to 205 of Patent Document 1

(Manufacturing Example 2)

The Mitsui Chemical database used in the present invention was prepared by the method described in paragraphs 0022 to 0030 of Patent Document 2

(Microbial strain)

All of MRSA (Methicillin-resistant Staphylococcus aureus):JMC16555 strain (catalog number), Escherichia coli:JCM1649T strains (catalog number), and Pseudomonas aeruginosa:JCM5962T strain (catalog number) used in the following examples are publicly available from RIKEN, BioResource Research Center, Microbe Division (JCM) (address: 305-0074, Takanodai 3-1-1, Tsukuba, Ibaraki, Japan).

(Example 1)

MRSA (Methicillin-resistant Staphylococcus aureus) was seeded on LB agar medium, incubated overnight at 30°C, and stored at 4°C after colony formation. Colonies were inoculated in 2 ml of LB liquid medium and incubated at 30°C, 180 rpm for 16 h with shaking. Cultures were measured for bacterial counts using a CytoFLEX (Beckman Coulter) to determine the bacterial concentration of the stock solution.

The stock solution of MRSA culture solution obtained above was diluted using 1×PBS to achieve bacterial counts of 100 CFU, 500 CFU, 1,000 CFU, 5,000 CFU, and 10,000 CFU per 200 μl. 200 μl of each diluted culture medium was added to Pathogen Lysis Tubes (Qiagen). In addition, 100 μl of Reagent DX, supplied with the product, and 400 μl of Lysis-/- Binding Buffer, supplied with the High Pure RNA Isolation Kit (Roche), were added and set in a Delta Mixer Se-08 (Titec) and vortexed for 10 minutes.

The column supplied with the High Pure RNA Isolation Kit (Roche) was set in a reservoir, and 550 μl of the supernatant of the above bacterial disruption solution was transferred to the column, and the column was centrifuged at 8,000 × g for 1 minute in a tabletop high speed micro centrifuge the CT15E (Hitachi Koki Co., Ltd.). After setting the column to a new reservoir, add 500 μl of Wash Buffer I supplied with the High Pure RNA Isolation Kit (Roche) and centrifuge for 1 min at 8000 × g. Again, the column was placed in a new reservoir and 500 μl of Wash Buffer II supplied with High Pure RNA Isolation Kit(Roche) was added and centrifuged for 1 min at 8000 × g. The column was placed in a new reservoir, and 200μl of Wash Buffer II supplied with High Pure RNA Isolation Kit(Roche) was added and centrifuged for 2 minutes at 13,000×g. The column was placed in a new 1.5ml Eppendorf tube, and 50 μl of Elution Buffer supplied with High Pure RNA Isolation Kit(Roche) was added, centrifuged at 8000 × g for 1 minutes, and RNA solution was collected.

10.75 μl of recovered RNA solution was mixed with 1 μl of primers for reverse transcription (consisting of the base sequence of SEQ ID NO:1) (invitrogen) at various concentrations from 0.005 μM to 100 μM were mixed and incubated at 70°C for 5 min, followed by 5 min incubation at 4°C. The final concentration of the primer for reverse transcription in the first reaction system for reverse transcription is 0.2nM to 4000nM. After incubation, mixtures of RNA with primers for reverse-transcription were supplemented with M-MLV Reverse Transcriptase, RNase (H-), 1 μl of M-MLV RT (H-) Point Mutant supplied with Point Mutant (Promega), 5 μl of M-MLV RT 5 × Reaction Buffer, 6.25 μl of dNTP (Nippongene), 0.2 μl of RNase Inhibitor Recombinant type(ToYoBo), and 0. 8 μl of 1×PBS to obtain the first reaction system (solution). The resulting first reaction system was incubated for 20 min at 50 ° C., thereby obtaining a liquid reaction mixture containing the synthesized cDNA.

PCR was performed using cDNA (2 μl) in the reaction mixture thus obtained as a template. The final concentration of the primer for reverse transcription, which was 0.2 nM-4000 nM at the time of the reverse transcription reaction, is 0.02 nM-400 nM in the second reaction system (solution) for PCR. The PCR solution as a second reaction system for PCR consists of 2μl of 10×Buffer (Nippon Gene), 2μl of 2. 0 mM CleanAMP ™Hot Start PCR (TriLink), 2μl of 25mM MgCl$_2$ (Nippon Gene), 1.2μl of primer pair (invitrogen) for 5μM PCR, 1μl of 1U/μl DNA polymerase (produced as described in paragraphs 195 to 205 of Patent Document 1), 1μl of Evagreen(Biotium), and 8.8μl of DW (Nacalai Tesque).

Note that the following primer pairs were individually used as primer pairs for PCR

    ▪ A primer pair 1a: a combination of a forward primer consisting of the base sequence of SEQ ID NO : 2 and a reverse primer consisting of the base sequence of SEQ ID NO : 3,

    ▪ A primer pair 2a: a combination of a forward primer consisting of the base sequence of SEQ ID NO : 4 and a reverse primer consisting of the base sequence of SEQ ID NO : 5,

    ▪ A primer pair 3a: a combination of a forward primer consisting of the base sequence of SEQ ID NO : 6 and a reverse primer consisting of the base sequence of SEQ ID NO : 7,

    ▪ A primer pair 4a: a combination of a forward primer consisting of a base sequence of SEQ ID NO : 8 and a reverse primer consisting of a base sequence of SEQ ID NO : 9,

    ▪ A primer pair 5a: a combination of a forward primer consisting of a base sequence of SEQ ID NO : 10 and a reverse primer consisting of a base sequence of SEQ ID NO : 11, ▪ A primer Pair 6a: a combination of a forward primer consisting of a base sequence of SEQ ID NO:12 and a reverse primer consisting of a base sequence of SEQ ID NO:13,

    ▪ A primer pair 7a: a combination of a forward primer consisting of a base sequence of SEQ ID NO : 14 and a reverse primer consisting of a base sequence of SEQ ID NO : 15

After preparation of the PCR solution, PCR tubes were set in a Rotor-Gene Q(Qiagen) and real-time PCR was performed. The reaction conditions for the PCR were 95° C., 5 minutes → 94° C., 10 seconds ▪ 60° C., 20 seconds ▪ 72° C., 10 seconds ▪ 85° C., and 2 seconds for 40 cycles → 95° C., 10 seconds. Bacteria were identified from the Tm values (melting temperature) of each PCR product obtained from the analyzed data by matching them to a Mitsui Chemicals database (prepared by the method described in paragraphs 0022 to 0030 of Patent Document 2). The results of the identification using the method described above are shown in Table 1. The evaluation in Table 1 was performed as follows.

○: Identifiable
Δ: Identifiable with dirty peaks
✕ : Not identifiable
- : Not tested

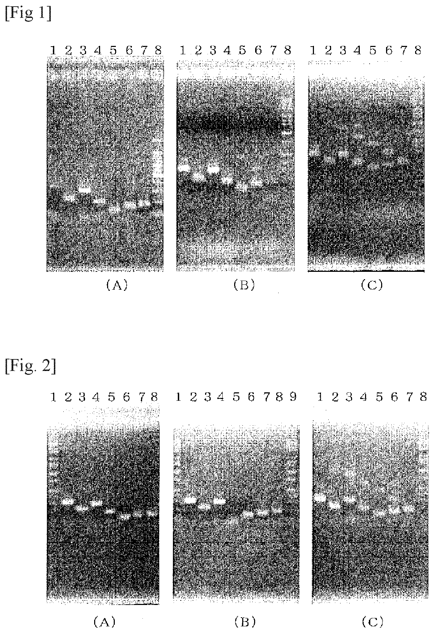

The results of agarose gel electrophoresis for 100 CFU PCR products at the added primer concentrations of 2, 5, and 10 μM are shown in Figure 1. Figure 1 (A) shows the results of agarose gel electrophoresis for PCR products at 2 μM of the added primers for reverse transcription. Figure 1(B) is the result of agarose gel electrophoresis of the PCR product at 5μM primer concentration for reverse transcription. Figure 1 (C) shows the results of agarose gel electrophoresis of PCR products at 10μM primer concentration for reverse transcription. Note that each lane denoted by the numbers in FIGS. 1 (A) to (C) shows the results of electrophoresis of the following products and compounds.

(1) A PCR-product of the primer-pair 1a
(2) A PCR-product of the primer-pair 2a
(3) A PCR-product of the primer-pair 3a
(4) A PCR-product of the primer-pair 4a
(5) A PCR-product of the primer-pair 5a
(6) A PCR-product of the primer-pair 6a
(7) A PCR-product of the primer-pair 7a
(8) A marker

[Table 1] **Table 1**

| S. aureus | | Upper row: Primer concentration of primer solution for reverse transcription (μM) Lower row : Concentration of primer for reverse transcription at the start of the DNA synthesis reaction by PCR (nM) | | | | | | | | | | | |
|---|---|---|---|---|---|---|---|---|---|---|---|---|---|
| | | 0.005 | 0.01 | 0.02 | 0.1 | 0.2 | 0.5 | 1 | 2 | 5 | 10 | 50 | 100 |
| | | 0.02 | 0.04 | 0.08 | 0.4 | 0.8 | 2 | 4 | 8 | 20 | 40 | 200 | 400 |
| **Number of bacteria (CFU/ Specimen)** | 100 | ✕ | ✕ | ✕ | ✕ | ✕ | ○ | ○ | ○ | ○ | ✕ | ✕ | ✕ |
| | 500 | - | - | ✕ | ○ | ○ | - | - | - | - | - | - | - |
| | 1000 | ✕ | ✕ | Δ | ○ | ○ | ○ | ○ | ○ | ○ | ✕ | ✕ | ✕ |
| | 5000 | - | - | ○ | ○ | ○ | - | - | - | - | - | - | - |
| | 10000 | - | - | ○ | ○ | ○ | ○ | ○ | ○ | ○ | ✕ | ✕ | ✕ |

As shown in Table 1, it was found that the ability of each bacterium to be identified depends on the concentration of primer for reverse transcription.
And, it was also proven that the identification was sufficiently sensitive even in the small fungus number by the proper adjustment of the reaction condition.

(Example 2)

E. coli was used to examine primer concentrations and bacterial counts. The same as in Example 1, except that the bacteria were set as E. coli. The identification results are shown in Table 2. The results of agarose gel electrophoresis are also shown in Figure 2. Figures 2(A)-(C) show the results of agarose electrophoresis at the added primer concentrations of 2, 5, and 10 μM in the primer solution for reverse transcription as in Figure 1. Note that each lane denoted by the numbers in Figures 2 (A) to (C) shows the results of electrophoresis of the following products and compounds.

- Figure 2 (A)

    (1) A marker
    (2) A PCR-product of the primer-pair 1a
    (3) A PCR-product of the primer-pair 2a
    (4) A PCR-product of the primer-pair 3a
    (5) A PCR-product of the primer-pair 4a
    (6) A PCR-product of the primer-pair 5a
    (7) A PCR-product of the primer-pair 6a
    (8) A PCR-product of the primer-pair 7a

- Figure 2 (B)

    (1) A marker
    (2) A PCR-product of the primer-pair 1a
    (3) A PCR-product of the primer-pair 2a
    (4) A PCR-product of the primer-pair 3a
    (5) A PCR-product of the primer-pair 4a
    (6) A PCR-product of the primer-pair 5a
    (7) A PCR-product of the primer-pair 6a
    (8) A PCR-product of the primer-pair 7a
    (9) A marker

- Figure 2 (C)

    (1) A PCR-product of the primer-pair 1a
    (2) A PCR-product of the primer-pair 2a
    (3) A PCR-product of the primer-pair 3a
    (4) A PCR-product of the primer-pair 4a
    (5) A PCR-product of the primer-pair 5a
    (6) A PCR-product of the primer-pair 6a
    (7) A PCR-product of the primer-pair 7a
    (8) A marker

[Table2]

| E. coli | | Upper row: Primer concentration of primer solution for reverse transcription ($\mu$ M) | | | | | | | | | | | |
| | | Lower row : Concentration of primer for reverse transcription at the start of the DNA synthesis reaction by PCR (n M) | | | | | | | | | | | |
| | | 0.005 | 0.01 | 0.02 | 0.1 | 0.2 | 0.5 | 1 | 2 | 5 | 10 | 50 | 100 |
| | | 0.02 | 0.04 | 0.08 | 0.4 | 0.8 | 2 | 4 | 8 | 20 | 40 | 200 | 400 |
| **Number of bacteria (CFU/ Specimen)** | 100 | × | × | × | Δ | ○ | - | ○ | ○ | ○ | × | × | × |
| | 500 | - | - | - | - | - | - | - | - | - | - | - | - |
| | 1000 | × | × | - | ○ | ○ | - | ○ | ○ | ○ | × | × | × |
| | 5000 | - | - | - | - | - | - | - | - | - | - | - | - |
| | 10000 | - | - | ○ | ○ | ○ | - | ○ | ○ | ○ | × | × | × |

As shown in Table 2, it was found that the ability of each bacterium to identify or not depends on the concentration of primer for reverse transcription.

And, it was also proven that the identification was sufficiently sensitive even in the small fungus number by the proper adjustment of the reaction condition.

(Example 3)

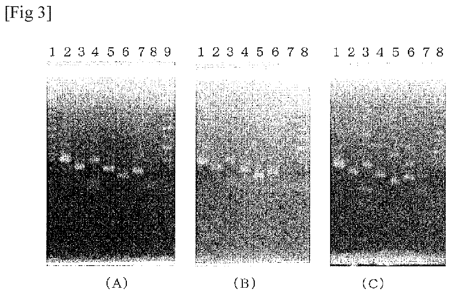

P. aeruginosa was used to examine primer concentrations and bacterial counts. The same as in Example 1, except that the bacteria were set as P. aeruginosa. The identification results are shown in Table 3. The results of agarose gel electrophoresis are also shown in Figure 3. Figures 3 (A)-(C) show the results of agarose electrophoresis at the added primer concentrations of 2, 5, and 10 $\mu$M in the primer solution for reverse transcription as in Figure 1. Note that each lane denoted by the numbers in Figures 3 (A) to (C) shows the results of electrophoresis of the following products and compounds.

- Figure 3 (A)

    (1) A marker
    (2) A PCR-product of the primer-pair 1a
    (3) A PCR-product of the primer-pair 2a
    (4) A PCR-product of the primer-pair 3a
    (5) A PCR-product of the primer-pair 4a
    (6) A PCR-product of the primer-pair 5a
    (7) A PCR-product of the primer-pair 6a
    (8) A PCR-product of the primer-pair 7a
    (9) A marker

- Fig. 3 (B) and (C).

    (1) A PCR-product of the primer-pair 1a
    (2) A PCR-product of the primer-pair 2a
    (3) A PCR-product of the primer-pair 3a
    (4) A PCR-product of the primer-pair 4a
    (5) A PCR-product of the primer-pair 5a
    (6) A PCR-product of the primer-pair 6a
    (7) A PCR-product of the primer-pair 7a
    (8) A marker

[Table 3] Table 3

| _P. aeruginosa_ | | Upper row: Primer concentration of primer solution for reverse transcription ($\mu$ M) | | | | | | | | | | | |
| | | Lower row : Concentration of primer for reverse transcription at the start of the DNA synthesis reaction by PCR (nM) | | | | | | | | | | | |
| | | 0.005 | 0.01 | 0.02 | 0.1 | 0.2 | 0.5 | 1 | 2 | 5 | 10 | 50 | 100 |
| | | 0.02 | 0.04 | 0.08 | 0.4 | 0.8 | 2 | 4 | 8 | 20 | 40 | 200 | 400 |
| **Number of bacteria (CFU/ Specimen)** | 100 | × | × | × | Δ | ○ | ○ | ○ | ○ | ○ | × | × | × |
| | 500 | - | - | - | - | - | | - | - | - | - | - | - |
| | 1000 | × | Δ | Δ | ○ | ○ | ○ | ○ | ○ | ○ | × | × | × |
| | 5000 | - | - | - | - | - | - | - | - | - | - | - | - |
| | 10000 | Δ | - | Δ | ○ | ○ | ○ | ○ | ○ | ○ | × | × | × |

As shown in Table 3, it was found that the ability of each bacterium to be identified depends on the concentration of primer for reverse transcription.
And, it was also proven that the identification was sufficiently sensitive even in the small fungus number by the proper adjustment of the reaction condition.

(Example 4)

MRSA used in Example 1 was used to confirm the sensitivity of this technique. Identification tests were performed as in Example 1, except that the number of bacteria per sample was set as 20 CFU and 40 CFU, with a primer solution for reverse transcription at a primer concentration of 1 μM, and the final concentration of the primer for reverse transcription in the second reaction system was 4 nM. For the primer pairs 1a to 7a, the obtained Tm values and identification results are shown in Table 4.

[Table 4]

**Table 4 : Sensitivity Identification Results**

| | Primer Pair No. | | | | | | | Identified Species |
|---|---|---|---|---|---|---|---|---|
| | **1a** | **2a** | **3a** | **4a** | **5a** | **6a** | **7a** | |
| 20 CFU-1 | 88.60 | 88.50 | 89.25 | 89.65 | 85.65 | 87.90 | 86.65 | *S. aureus* |
| 20 CFU-2 | 88.75 | 88.60 | 89.25 | 89.65 | 85.65 | 87.75 | 86.50 | *S. aureus* |
| 20 CFU-3 | 88.50 | 88.50 | 89.15 | 89.60 | 85.75 | 87.75 | 86.50 | *S. aureus* |
| 40 CFU-1 | 88.40 | 88.50 | 89.25 | 89.65 | 85.75 | 87.65 | 86.75 | *S. aureus* |
| 40 CFU-2 | 88.40 | 88.40 | 89.15 | 89.50 | 85.65 | 87.65 | 86.50 | *S. aureus* |

INDUSTRIAL APPLICABILITY

The method of the present invention enables rapid detection and identification of causative organisms of an infectious disease, especially of sepsis, and realizes a rapid diagnostic method for sepsis.

In other words, the present invention makes it possible to construct a system for identification of causative organisms by a single PCR, thereby reducing the workload and enabling full automation of testing.

**Claims**

1. A bacterial identification method **characterized by** having the following steps (1) to (3):

   (1) A step of performing a reverse transcription reaction using a first reaction system comprising a primer for reverse transcription to prepare a cDNA containing a base sequence for identification of a bacterium of interest, an RNA extracted from the bacterium in a sample, and an enzyme with RNA-dependent DNA polymerase activity to obtain a reaction mixture containing the synthesized cDNA and the primers for the reverse transcription,
   (2) A step of performing PCR using a second reaction system comprising the reaction mixture, a primer pair for synthesis of a double-stranded DNA containing the base sequence for identification of the bacterium of interest, and an enzyme with DNA-dependent DNA polymerase activity, provided that in the second reaction system, the concentration of the primer for reverse transcription supplied from the reaction mixture is not less than 0.08nM and not more than 20nM, and
   (3) A step of detecting the generation of the double-stranded DNA containing the base sequence for identification of the bacterium of interest from the second reaction system after PCR.

2. The bacterial identification method according to claim 1, **characterized in that** the steps (1) and (2) are performed sequentially in different reaction vessels or in the same reaction vessel.

3. The bacterial identification method according to claim 1 or 2, **characterized in that** the basic sequence for identification of the bacterium of interest is a basic sequence contained in a 16S rDNA of the bacterium and the RNA includes a 16S rRNA of the bacterium in the sample.

4. The bacterial identification method according to claim 3, **characterized in that** the primer for reverse transcription consist of a base sequence of SEQ ID NO : 1.

5. The bacterial identification method according to claim 3 or 4, **characterized in that** the primer pair is at least one primer pair selected from the group consisting of the following (Group A) to (Group C):

   (Group A)

   - A primer pair 1a: a combination of a forward primer consisting of a base sequence of SEQ ID NO : 2 and a reverse primer consisting of a base sequence of SEQ ID NO : 3,
   - A primer pair 2a: a combination of a forward primer consisting of a base sequence of SEQ ID NO : 4 and a reverse primer consisting of a base sequence of SEQ ID NO : 5,
   - A primer pair 3a: a combination of a forward primer consisting of a base sequence of SEQ ID NO : 6 and a reverse primer consisting of a base sequence of SEQ ID NO : 7,
   - A primer pair 4a: a combination of a forward primer consisting of a base sequence of SEQ ID NO : 8 and a reverse primer consisting of a base sequence of SEQ ID NO : 9,
   - A primer pair 5a: a combination of a forward primer consisting of a base sequence of SEQ ID NO : 10 and a reverse primer consisting of a base sequence of SEQ ID NO : 11,
   - A primer Pair 6a: a combination of a forward primer consisting of a base sequence of SEQ ID NO:12 and a reverse primer consisting of a base sequence of SEQ ID NO:13,
   - A primer pair 7a: a combination of a forward primer consisting of a base sequence of SEQ ID NO : 14 and a reverse primer consisting of a base sequence of SEQ ID NO : 15

   (Group B)
   A primer pair in which one or two bases have been added, deleted or replaced in part of the sequence of one or both primers of each primer pair in (group A) above without impairing their function as primers, and
   (Group C)
   A primer pair consisting of a complementary sequence corresponding to the base sequence of one or both primers of each primer pair in (Group A) or (Group B) above.

6. The bacterial identification method according to any one of claims 1 to 5, **characterized in that** in the step (3), Tm value of the generated double-stranded DNA is measured, and the obtained Tm value is compared with the Tm value of the double-stranded DNA including the sequence for identification of the bacterium of interest that has been measured beforehand, or data obtained secondarily from them, to identify the bacterium in the sample.

7. The bacterial identification method according to any one of claims 1 to 6, **characterized in that**, after the step (1), the reaction mixture is directly alone or diluted and used in the step (2).

8. A kit for identifying bacteria in a sample by PCR using as a template a cDNA contained in a reaction mixture by reverse transcription reaction of an RNA extracted from bacteria in the sample, **characterized in that** the kit comprising (a) to (c) below:

    (a) a primer for reverse transcription to prepare a cDNA containing a base sequence for identification of the bacterium of interest for preparation of the first reaction system for reverse transcription reaction,
    (b) a primer pair for synthesis of a double-stranded DNA containing the base sequence for the identification of the bacteria of interest, for the preparation of a second reaction system for the PCR, and
    (c) an enzyme with RNA-dependent DNA polymerase activity,

    wherein, the second reaction system comprises the reaction mixture by the reverse transfer reaction, wherein the amount of primer for reverse transcription is adjusted such that the concentration of the primer for reverse transcription when fed through the reaction mixture to the second reaction system is 0.08nM or more and 20nM or less.

9. The kit according to claim 8, **characterized in that** further comprising an instruction manual describing the reverse transcription reaction and the PCR method.

10. The kit according to claim 9, **characterized in that** the instruction manual describes the procedure so that the concentration of the primer for reverse transcription in the second reaction system is not less than 0.08 nM and not more than 20 nM.

11. The kit according to any one of claims 8 to 10, **characterized in that** the basic sequence for identification of the bacterium of interest is a basic sequence contained in a 16S rDNA of the bacterium and the RNA includes a 16S rRNA of the bacterium in the sample.

12. The kit according to claim 11, **characterized in that** the primer for reverse transcription consist of a base sequence of SEQ ID NO : 1.

13. The kit according to claim 11 or 12, **characterized in that** the primer pair is at least one primer pair selected from the group consisting of the following (Group A) to (Group C):

    (Group A)

    - A primer pair 1a: a combination of a forward primer consisting of a base sequence of SEQ ID NO : 2 and a reverse primer consisting of a base sequence of SEQ ID NO : 3,
    - A primer pair 2a: a combination of a forward primer consisting of a base sequence of SEQ ID NO : 4 and a reverse primer consisting of a base sequence of SEQ ID NO : 5,
    - A primer pair 3a: a combination of a forward primer consisting of a base sequence of SEQ ID NO : 6 and a reverse primer consisting of a base sequence of SEQ ID NO : 7,
    - A primer pair 4a: a combination of a forward primer consisting of a base sequence of SEQ ID NO : 8 and a reverse primer consisting of a base sequence of SEQ ID NO : 9,
    - A primer pair 5a: a combination of a forward primer consisting of a base sequence of SEQ ID NO : 10 and a reverse primer consisting of a base sequence of SEQ ID NO : 11,
    - A primer Pair 6a: a combination of a forward primer consisting of a base sequence of SEQ ID NO:12 and a reverse primer consisting of a base sequence of SEQ ID NO:13,
    - A primer pair 7a: a combination of a forward primer consisting of a base sequence of SEQ ID NO : 14 and a reverse primer consisting of a base sequence of SEQ ID NO : 15

    (Group B)
    A primer pair in which one or two bases have been added, deleted or replaced in part of the sequence of one or both primers of each primer pair in (group A) above without impairing their function as primers, and
    (Group C)
    A primer pair consisting of a complementary sequence corresponding to the base sequence of one or both primers of each primer pair in (Group A) or (Group B) above.

[Fig 1]

(A)  (B)  (C)

[Fig. 2]

(A)  (B)  (C)

[Fig 3]

1 2 3 4 5 6 7 8 9     1 2 3 4 5 6 7 8     1 2 3 4 5 6 7 8

(A)         (B)         (C)

<table>
<tr><td colspan="2" align="center">**INTERNATIONAL SEARCH REPORT**</td><td colspan="2">International application No.<br>PCT/JP2019/013021</td></tr>
</table>

**A. CLASSIFICATION OF SUBJECT MATTER**
Int.Cl. C12Q1/689(2018.01)i, C12N15/11(2006.01)n

According to International Patent Classification (IPC) or to both national classification and IPC

**B. FIELDS SEARCHED**

Minimum documentation searched (classification system followed by classification symbols)
Int.Cl. C12Q1/689, C12N15/11

Documentation searched other than minimum documentation to the extent that such documents are included in the fields searched
Published examined utility model applications of Japan 1922–1996
Published unexamined utility model applications of Japan 1971–2019
Registered utility model specifications of Japan 1996–2019
Published registered utility model applications of Japan 1994–2019

Electronic data base consulted during the international search (name of data base and, where practicable, search terms used)
CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2010-162042 A (UNIVERSITY OF PITTSBURGH OF THE COMMONWEALTH SYSTEM OF HIGHER EDUCATION) 29 July 2010, claims, paragraphs [0002], [0029], example 1 & WO 2002/070751 A1, claims, paragraphs [0002], [0047], example 1 & US 2003/0017482 A1 & CN 1500151 A | 1-13 |
| A | JP 2018-011590 A (QVELLA CORPORATION) 25 January 2018 & US 2014/0004501 A1 & WO 2013/013304 A1 | 1-13 |

☒ Further documents are listed in the continuation of Box C.　　☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>18 June 2019 (18.06.2019) | Date of mailing of the international search report<br>25 June 2019 (25.06.2019) |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**EP 3 778 925 A1**

**INTERNATIONAL SEARCH REPORT**

| | International application No. |
|---|---|
| | PCT/JP2019/013021 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2012-034705 A (E.I. DUPONT DE NEMOURS AND COMPANY) 23 February 2012 & US 2005/0266468 A1 & WO 2006/085906 A2 | 1-13 |
| A | DRESES-WERRINGLOER, U. et al., "Presistence of Chlamydia trachomatis is induced by ciprofloxacin and olfloxacin in vitro", Antimicrob. Agents Chemother., 2000, vol. 44, no. 12, pp. 3288-3297 | 1-13 |
| A | WO 2015/053293 A1 (MITSUI CHEMICALS, INC.) 16 April 2015 & US 2016/0244812 A1 & CN 105612254 A | 1-13 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010082640 A
- WO 2007097323 A
- JP 2012034705 A
- WO 2010054819 A
- WO 2015053293 A

**Non-patent literature cited in the description**

- **S. CHAKRAVORTY et al.** *Journal of Microbiological Methods,* 2007, vol. 69, 330-339

SEQUENCE LISTING
<110>  Mitsui Chemicals, inc.
<120>  A method of determining a microorganism using RNA of a bacterial sample

<130>  18-0076-MCI

<150>  JP 2018-058663
<151>  2018-3-26

<160>  15

<210>  1
<211>  18
<212>  DNA
<213>  Artificial

<220>
<223>  Primer for Reverse Transcription

<400>  1
agacccggga acgtattc                    18


<210>  2
<211>  23
<212>  DNA
<213>  Artificial

<220>
<223>  Forward primer for PCR

<400>  2
gcaggcttaa cacatgcaag tcg              23


<210>  3
<211>  18
<212>  DNA
<213>  Artificial

<220>
<223>  Reverse Primer for PCR
<400>  3
cgtaggagtc tggaccgt                    18


<210>  4
<211>  19
<212>  DNA
<213>  Artificial

<220>
<223>  Forward primer for PCR

<400>  4
gtccagactc ctacgggag                   19


<210>  5
<211>  17
<212>  DNA
<213>  Artificial

21

```
<220>
<223>  Reverse Primer for PCR

<400>  5
cctacgtatt accgcgg                         17


<210>  6
<211>  16
<212>  DNA
<213>  Artificial

<220>
<223>  Forward primer for PCR

<400>  6
agcagccgcg gtaata                          16


<210>  7
<211>  23
<212>  DNA
<213>  Artificial

<220>
<223>  Reverse Primer for PCR

<400>  7
ggactaccag ggtatctaat cct                  23


<210>  8
<211>  23
<212>  DNA
<213>  Artificial

<220>
<223>  Forward primer for PCR

<400>  8
aacaggatta gataccctgg tag                  23


<210>  9
<211>  21
<212>  DNA
<213>  Artificial

<220>
<223>  Reverse Primer for PCR

<400>  9
aattaaacca catgctccac c                    21


<210>  10
<211>  21
<212>  DNA
<213>  Artificial

<220>
<223>  Forward primer for PCR
```

```
<400>  10
tggtttaatt cgatgcaacg c                          21


<210>  11
<211>  17
<212>  DNA
<213>  Artificial

<220>
<223>  Reverse Primer for PCR

<400>  11
gagctgacga cagccat                               17


<210>  12
<211>  18
<212>  DNA
<213>  Artificial

<220>
<223>  Forward primer for PCR

<400>  12
gttaagtccc gcaacgag                              18


<210>  13
<211>  22
<212>  DNA
<213>  Artificial

<220>
<223>  Reverse Primer for PCR

<400>  13
ccattgtagc acgtgtgtag cc                         22


<210>  14
<211>  22
<212>  DNA
<213>  Artificial

<220>
<223>  Forward primer for PCR

<400>  14
ggctacacac gtgctacaat gg                         22

<210>  15
<211>  18
<212>  DNA
<213>  Artificial

<220>
<223>  Reverse Primer for PCR

<400>  15
agacccggga acgtattc                              18
```